# EUROPEAN PATENT SPECIFICATION

(11) **EP 3 342 451 B1**
(45) Date of publication and mention of the grant of the patent: **24.06.2020**
(21) Application number: 17166803.1
(22) Date of filing: 18.04.2017
(51) Int. Cl.: A61N 1/04

(54) **ELECTROTHERAPEUTICAL DEVICE**
ELEKTROTHERAPEUTISCHE VORRICHTUNG
DISPOSITIF ÉLECTROTHERAPEUTIQUE

(30) Priority: 28.12.2016 JP 2016254601
(43) Date of publication of application: 04.07.2018
(73) Proprietor: Minato Medical Science Co., Ltd., Osaka 532-0025 (JP)
(72) Inventor: SASAOKA, Hiromasa, Koube-sity, Hyogo 650-0047 (JP)
(74) Representative: Gunzelmann, Rainer

(56) References cited:
- EP-A1- 0 289 905
- JP-A- 2000 342 695
- JP-A- 2003 190 301
- US-A- 3 640 270

## Description

### TECHNICAL FIELD

The present invention relates to an electrotherapeutic device.

### BACKGROUND ART

There are many kinds of electrical stimulation therapies. These are classified as therapeutic electrical stimulation, percutaneous electrical stimulation, and functional electrical stimulation. Furthermore, percutaneous electrical stimulation is subdivided into low frequency therapy, interference low frequency therapy, and the like.

Generally, low frequency therapy is known to have the anti-inflammatory analgesic effects and treatment frequency is generally 0.1 - 999 Hz, preferably 0.1-199Hz.

Therefore, it is widely used in the fields of orthopedic surgery, physiotherapy and the like. An electrotherapeutic device gives stimulation to the muscles of the user. Therefore, it is expected to expand the filed into improvement of user's physical function and muscle strength as well as the anti-inflammatory analgesic effects.

Generally, an electrotherapeutic device gives an electrical stimulation. For this purpose, we use a conductor being also called an electrode. The conductor is attached to the treatment portion by a band or the like. However, it is often dislocated from its correct position on the ground of a user's movement. On the contrary, if you force a user not to move, they will probably feel uneasy. Additionally, a user has so many body parts with different shapes that it is difficult to stick without displacement on every part of the body. It also depends on the attaching skills of the operator. Furthermore, the more carefully it is attached, the more time it needs.

Additionally, if the conduction state gets worse for some reason, the device itself or a practitioner may try to improve in order to return it to its appropriate states. However, after that, if it unexpectedly returns to its appropriate states, there is a possibility to supply excessive voltage or current instantaneously. For this reason, in order to ensure stable conduction in the electrotherapy, there are various kinds of conductors depending on the treatment part of the body, treatment method, function, purpose to use, and the like.

In order to get a stable contacting state, there are some types of conductors. As one of them, for example, a wet sponge in a flexible cup is often used at the conductive portion. In this case, the atmospheric pressure inside the cup is kept lower than the outside to be attached firmly.

A sticky type conductor is also used as a conventional conductor. The predetermined adhesive strength at the beginning tends to drop when it is stuck again. In addition, the body surface condition causes the contacting state.

A conventional conductor attached to the user's body surface by deforming the flexible cup to make an air pressure inside of the cup lower than outside is disclosed in the prior art document 1 (JP-A-2000-342695, Fig. 11).

The suction force of this prior art is so weakened by the body movement that it causes conduction failure or removal of the conductor.

Another conventional conductor is also disclosed in the prior art document 2. (JP-A-2003-190301, FIG. 12) The inside of the flexible cup is sucked with a suction pump to attach the conductor to the body surface. Therefore, it is stable as for suction force if there are some body movements.

However, especially in the winter, the wet sponge inside the flexible cup is cold to attach to the body surface. In order to solve this problem, a warming means (for example, a hot plate) is provided on the main body for preliminarily heating.

When the conductors are on the hot plate, the conductors are warm enough. However, when removing the conductors from the hot plate, the conductors are quickly cooled down due to sucking the water in the sponge.

Also, when sucking the inside of the cup, unnecessary substances such as dust, scraps separated from the sponge, perspiration, sebum and hand cream adhering to the body surface are simultaneously sucked. As a result, clogging due to the unnecessary material may occur in the flow path of the suction guide cord and the suction pump, and it may significantly reduce the suction force.

EP 0 289 905 A1 concerns a suction electrode, consisting of an electrode housing in which an electrically conducting electrode and a low-pressure nozzle are arranged, the low-pressure nozzle having a channel which is, at least at one position, designed narrower than at other positions in order to produce a low pressure upon passing through of a medium, and is provided with a suction channel opening at the level of the narrow position.

US 3,640,270 concerns a contactor for diagnostic or therapeutic use on human or animal tissue comprises a suction cup of elastomeric material containing an electrolyte-impregnated sponge near the mouth of the cup supported on an electrode plate from which a conductor extends insulatedly outwardly inside a tube leading to a source of air under pressure.

### SUMMARY OF THE INVENTION

The following presents a general summary of aspects of the invention in order to provide a basic understanding of the invention. This summary is not an extensive overview of the invention. It is not intended to delineate the scope of the invention. The following summary merely presents some concepts of the invention in a general form as a prelude to the more detailed description provided below.

The present invention is intended to solve the above-described problems and to provide an electrotherapeutic device which can keep a warmed conductor and suppress unnecessary clogging of the suction guide cord or the pump.

In order to solve the above problem, the present electrotherapeutic device comprises the features of claim 1.

The present invention prevents from cooling the containing conductive liquid of the absorber during treatment, and clogging in the suction guide cord or the suction pump with fragments separated from the absorber and unnecessary substances such as perspiration and sebum which have adhered to the user's body surface.

The present invention is set out in the appended claims. The embodiments, examples or aspects according to the present description which do not fall within the scope of said claims are provided for illustrative purposes only and do not form part of the present invention.

### BRIEF DESCRIPTION OF THE DRAWINGS

To allow a better understanding of the present invention, embodiments will now be described by way of example, with reference to the accompanying drawing in which:
FIG. 1a 1b are external views of the conductor according to the embodiment 1;
FIG. 1c is a cross sectional view of the conductor according to the embodiment 1;
FIG. 2 is an external view of each component of the conductor according to the embodiment 1;
FIG. 3 is a cross sectional view of the conductor according to the example 1;
FIG. 4a 4b are external views of the conductor according to the example 2;
FIG.4c is a cross sectional view of the conductor according to the example 2;
FIG. 5a 5b 5c are external views of the conductor according to the example 3;
FIG.5d is a cross sectional view of the conductor according to the example 3;
FIG. 6a 6b are external views of the conductor according to the example 4;
FIG.6c is a cross sectional view of the conductor according to the example 4;
FIG. 7a 7b 7d are cross sectional views of the conductor according to the example 5 5;
FIG. 7c is an external view of the conductor according to the example 5;
FIG. 8 is an explanatory view showing the temperature change of the conductor according to the embodiment 1;
FIG. 9 is a characteristic diagram showing a current density of a conductor according to the embodiment 1;
FIG. 10a is an external view of an electrotherapeutic device for operating the conductor according to the embodiment 1;
FIG. 10b is a block diagram of an electrotherapeutic device for operating the conductor according to the embodiment 1;

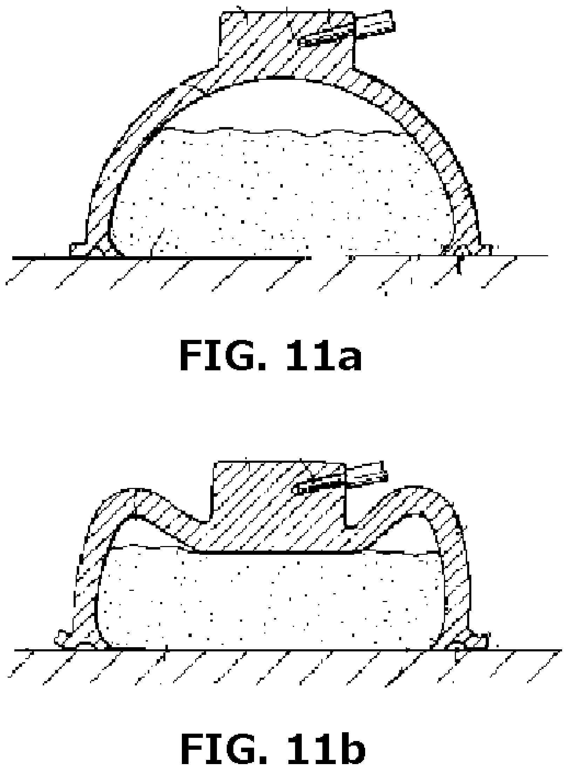

FIG. 11 is a cross sectional view of the conductor according to a conventional technique;
FIG. 12a is an external view of the conductor of an electric therapy device for operating the conductor according to another conventional technique;
FIG. 12b is a cross sectional view of the conductor according to another conventional technique;
FIG. 12c is a block diagram of an electric therapy device for operating the conductor according to another conventional technique;
FIG. 13 is a cross sectional view of the adsorption apparatus according to another conventional technique.

### DETAILED DESCRIPTION

### Embodiment 1

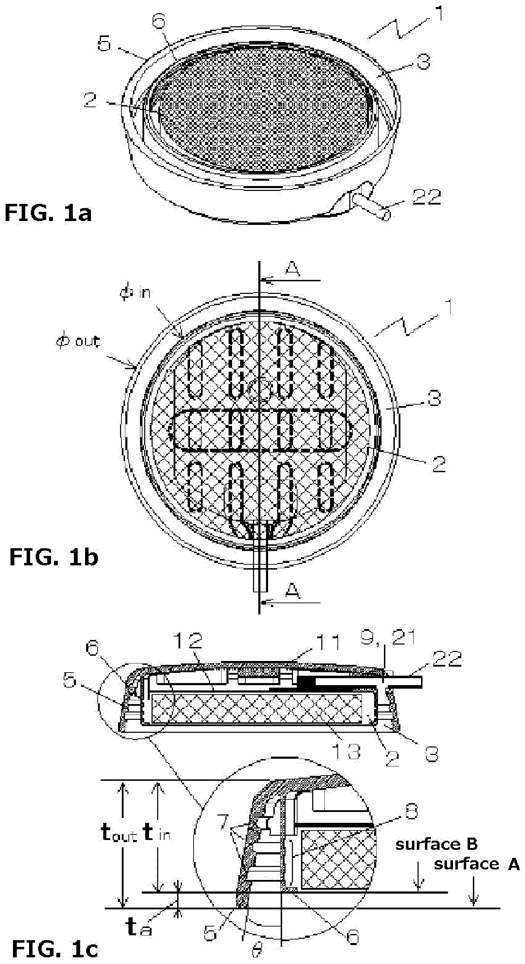

Hereinafter, a first embodiment is described with reference to FIG. 1.

The conductor 1 includes an outer cover 5 having a cup-shaped portion, an inner cover 6 having a cup-like portion and disposed inside the outer cover 5, a conductive portion 2 disposed inside the inner cover 6, and a suction portion 3 disposed between inside the outer cover 5 and outside the inner cover 6. (See FIG. 1a). The outer cover 5 is preferably disposed all around the inner cover 6 to suction uniformly.

The cup-shaped portion of the outer cover 5 and the inner cover 6 composes an absorber having flexibility and is deformed according to the body surface of the user.

The contacting portion of the cup-shaped portion of the outer cover 5 can be shaped according to the contacting area of the user, but it is possible to adopt a flat shape surface to fit for many body portions.

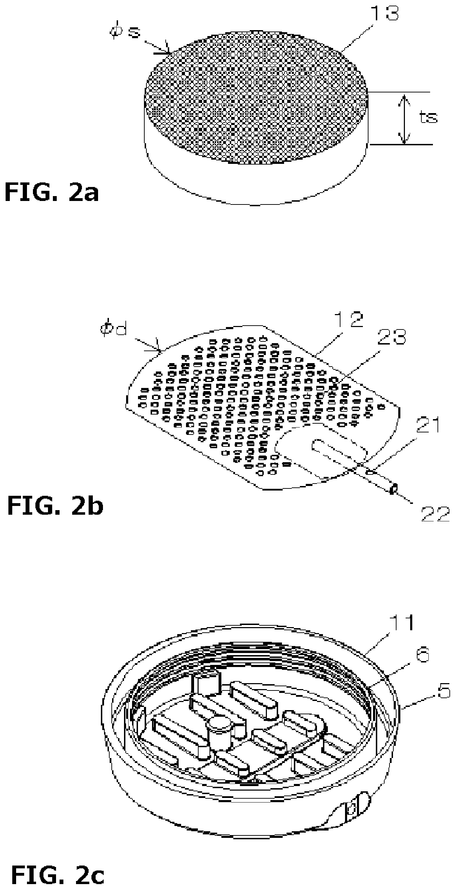

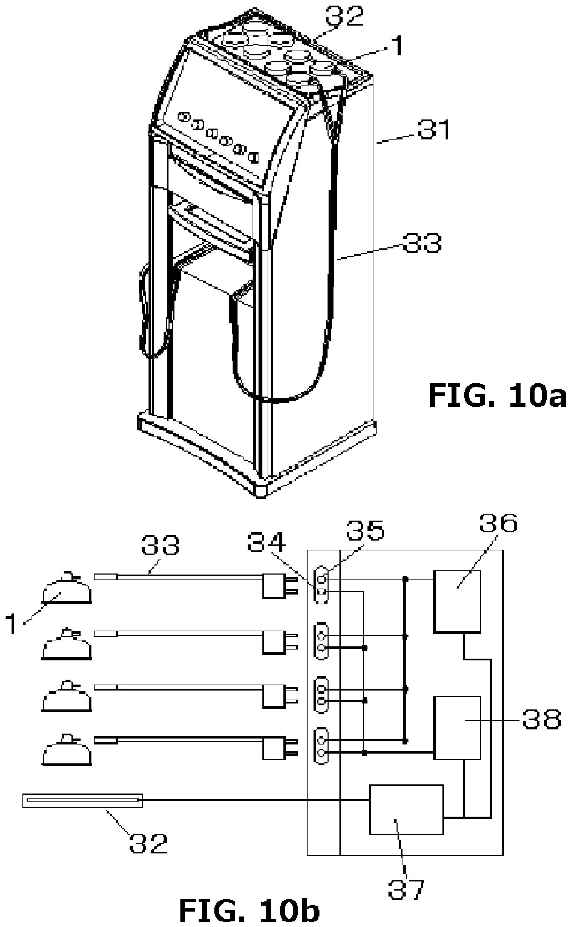

In addition, the conductive portion 2 has the absorber 13 containing conductive liquid (including water) and an electrode plate 12. (See FIG. 2) By placement on the warming means 32 (for example, a hot plate) of the main body 31 (see FIG. 10), the conductor 1 is warmed up.

During treatment, the practitioner picks up the conductor 1 from the warming means 32 and attaches it to the user's body surface.

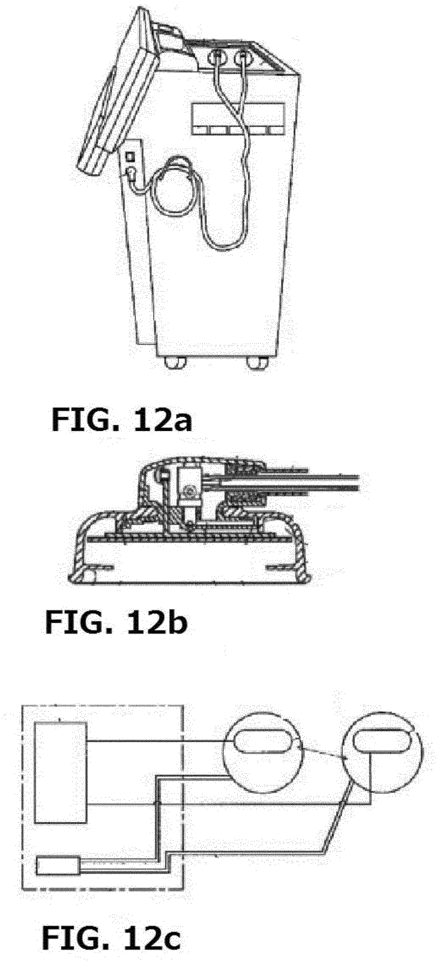

As for the conventional conductor (see FIG. 12b), it is rapidly cooled down during the treatment. It is mainly caused by suctioning the containing conductive liquid of the absorber 13.

Therefore, as for this invention, the absorber 13 is placed inside the inner cover 6. As a result, the absorber 13 inside the inner cover 6 is neither sucked nor unexpectedly cooled during the treatment.

Further, the conventional conductor cannot keep warm even if a warming function such as a heater is provided inside the conductor due to the sucking through the sponge containing the conductive liquid.

In this respect, this embodiment can be kept warm more effectively when a heater is provided inside the inner cover 6, for example, on the surface of the electrode plate 12.

Furthermore, since the inside of the inner cover 6 is not actively sucked during treatment, it can suppress the suction of impurities from the absorber 13.

In this embodiment, the tip of the contacting portion of the inner cover 6 is shorter than that of the outer cover 5, in other words, farther away from the body surface of the user. (See FIG. 1c).

When attaching the conductor 1 to the body surface, the tip of the contacting portion of the outer cover 5 contacts first with the body surface. At this moment, since the tip of the contacting portion of the inner cover 6 does not contact with the body surface, the water contained in the absorber 13 can be suctioned a little. However, as the tip of the contacting portion of the inner cover 6 will contact with the body surface promptly, the water is hardly suctioned at this moment.

If the opposite tip position arrangement is used, when attaching the conductor 1 to the body surface, the tip of the contacting portion of the inner cover 6 reaches at the body surface before that of the outer cover 5 reaches. Therefore, a gap formed between the body surface and the tip of the contacting portion of the outer cover 5 causes the leak of air when sucking.

In order to eliminate the gap between them, the practitioner has to press the outer cover 5 by stronger force. Further, in this case, even after being attached to the body surface, it can be separated due to the reaction force at the tip portion of the contacting portion of the inner cover 6.

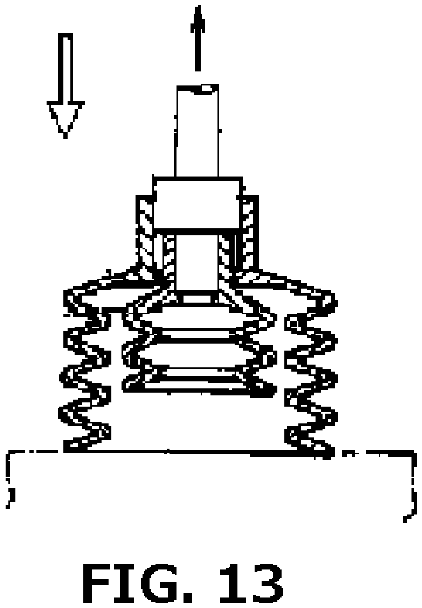

Furthermore, by providing a recess or a slit 7 inside of the outer cover 5 and/or the inner cover 6 (see FIG.1c), without forming a conventional bellows structure (see FIG. 13), the cup can be deformed effectively toward the body surface.

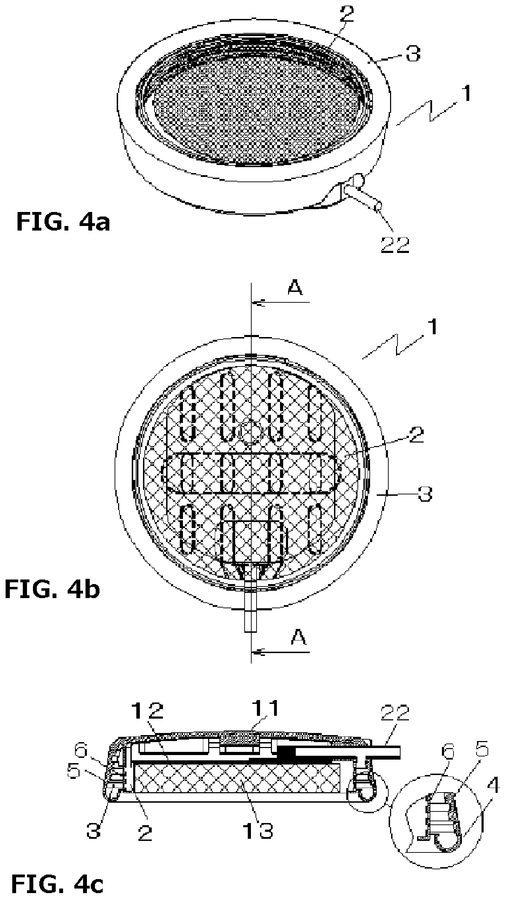

Additionally, buffer portions 4 are provided at both tip portions of the inner cover 6 and the outer cover 5. (See FIG.4c). Attaching the conductor to the body surface may result in scarring due to congestion. The scar may remain on the skin for a long time. In addition, when the temperature of the body surface is low, the symptoms become more prominent. On that point, this embodiment can keep the conductor 1 warmer so that it can be expected to reduce the scar due to congestion.

If the buffering portion 4 is wound inwardly, it is easy to follow the body surface during adsorption and it is possible to suppress peel of the conductor from the body surface.

Further, the electrode plate 12 is provided with fine holes 23. On the other hand, a plurality of ribs are provided on the inner bottom surface of the cup 11 (inner cover 6, see FIG. 2). The ribs are facing to the plurality of holes of the electrode plate 12. As for a conventional conductor, the fine holes 23 are provided for increasing the elasticity of the electrode plate 12 and the plurality of ribs are provided for making it easier to deform the cup in a direction parallel to the ribs and to make a space between the cup and the electrode plate during sucking in order to prevent the sponge from being pushed into the suction port.

This embodiment also has a similar structure, but the role thereof is different from a conventional one. Specifically, the absorber 13 is compressed by contacting with the body surface, and the conductive liquid contained therein is discharged to the outside of the absorber 13. So, the space between the cup and the electrode plate is used as the conductive liquid retention space.

In addition, the conductive liquid in the sponge (absorber) has to be frequently replenished, as with an conventional conductor, but this embodiment can suppress the conductive liquid reduction of the absorber 13 effectively.

### Example 1

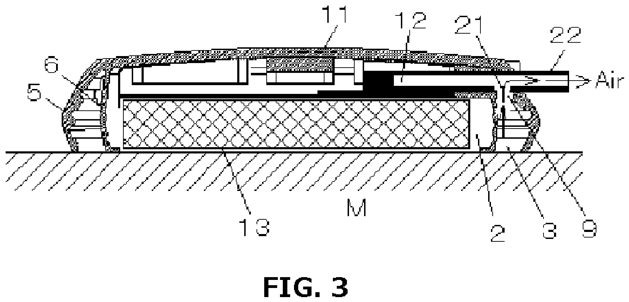

Hereinafter, an example is described. FIG. 1a is an external perspective view, as seen from the side where the suction guide 1 is attached to a user, FIG.1b is a front view thereof, FIG.1c is a cross sectional view of the conductor, FIG. 2a is a perspective view of the outer appearance of the absorber 13, FIG. 2b is a perspective view of the appearance of the electrode plate 12, FIG. 2c is a perspective view of the appearance of the cup 11, FIG. 3 is a cross sectional view showing a state when the conductor 1 is operated, and FIG.10 is an external view and a block diagram of the electrotherapeutic device.

The main body 31 includes a suction pump 38 for generating a suction force of the conductor 1 and warming means 32 (hot plate or the like) for preliminarily heating the conductor 1.

The conductor 1 of this example has a conductive portion 2 (mainly occupied by absorber 13), a suction portion 3, an outer cover 5, an inner cover 6, an absorber 13, and an electrode plate 12. (See FIG.1a).

Each material differs depending on the purpose of use. Specifically, the material of the outer cover 5 and the inner cover 6 should be determined on the premise of a plurality of uses, and the absorber should have durability.

The cup 11 directly affects the attaching force of the conductor 1 to the body surface. When it is attached to the body surface, it is deformed by the suction force. For this reason, the cup 11 is preferably made of a polymer material having flexibility and which hardly causes plastic deformation for a long time.

Also, the hardness of the cup 11 affects the sticking performance to the body surface. In this example, the hardness HS (shore A) is designed at 30 ° to 60 ° in consideration of sticking performance and versatility. On the other hand, the hardness of the cup 11 also affects the strength of scarring occurring to the user. In this example, a cushioning structure is adopted for the adsorbing portion, but in order to suppress the influence of scarring, it is possible to design all or part of the cup with an absorber having the hardness HS (shore A) of 30 ° or less.

Also, depending on the therapeutic application and the treatment body portion, several cups 11 having some optimum shape and size may be prepared in advance.

The suction portion 3 is constituted by the inside of the outer cover 5 and the outside of the inner cover 6. The suction portion 3 is formed in a truncated cone shape so as not to be partially closed between the outer cover 5 and the inner cover 6, depending on the suction force or attachment state.

Specifically, as shown in FIG. 1c, the wall forming the inner cover 6 is provided substantially vertically, and the wall forming the outer cover 5 is provided with an angle θ.

If the angle θ is too large, the area of the surface for attaching becomes too large as compared with the area of the surface for sucking. As a result, when using a plurality of conductors 1, the attachment location may overlap the neighboring conductor. Also, a large attachment area makes it more difficult to follow the curved surface of the user's body.

On the other hand, if the area of the surface for attaching is kept unchanged and the area of the surface for sucking is made smaller, the current density of the treatment is a possibility to cause skin damage or burn on the affected part to which current is applied.

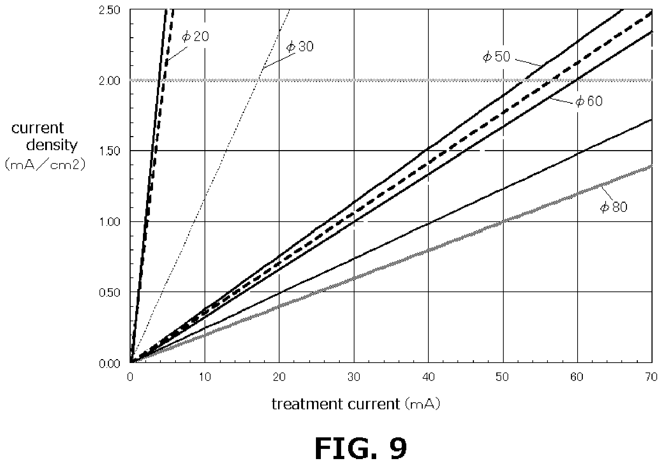

In FIG. 9, the current density is plotted when the area of the surface for sucking (diameter Φ ᵢₙ) is changed with the treatment current being plotted on the abscissa and the current density being plotted on the ordinate.

The current density is obtained by dividing the treatment current by the surface area for sucking, and it is in inverse relation to the surface area for sucking. The current density is preferably suppressed to 2.0 mA / cm² or less in order not to cause the aforementioned skin damage and burn injury.

The electrical stimulating therapy promotes blood circulation to raise the body temperature and sweat. As a result, the state of electrical contact between the conductive portion 2 and the body surface can become locally better to make the local treatment current larger unexpectedly. In order to prevent this, we need to select proper material of the absorber 13 to contain conductive liquid sufficiently in order not to change the electrical contact state between the conductive portion 2 and the body surface.

In addition, the current density is also affected by the performance of sticking to the body surface. When the sticking is weak and partially, the current density increases unexpectedly.

Furthermore, if a problem occurs in the suction state between the body surface and the conductor 1 due to the sticking condition, the recovering performance of the conductor 1 is affected by the exhaust flow rate of the suction pump 38. The exhaust flow rate is preferably 5 L / min in this example.

The performance of the suction pump 38 is specified with the suction pressure [kPa] and exhaust flow rate shown by a characteristic graph. In order to suck without no difficulty, the VA value (= suction pressure [kPa] multiplied by exhaust flow rate [L / min]) is at least 100 or more. On the other hand, if the suction pressure is too high, scarring tends to remain on the body surface, and it is difficult to detach the conductor 1. Considering these balances, the VA value = 1000 is considered as the upper limit.

In case of using the pump 38 having the above-mentioned VA value, it is appropriate that the angle θ is 45 ° or less, and especially in this example, the angle θ is preferably set 5 to 15 °. (See FIG. 1c).

The electrode plate 12 is electrically connected to the main body 31 via the suction guide cord 33. In this example, we use a lead-out pipe 22 which forms a suction path by using lateral hole 21. In addition, since the electrode plate 12 is in contact with the containing conductive liquid of the absorber 13 for a long time, a material with conductive liquid resistant performance, for example SUS, is ideal to use.

Since the lead-out pipe 22 is also an electrical contact point to the electrode plate 12, it is preferable to use phosphor bronze, brass or the like and further plated. The lead-out pipe 22 is provided so as to penetrate the cup 11 and electrically connected to the electrode plate 12 by welding or caulking or the like. The electrode plate 12 is provided in a circular shape and its diameter Φ _{d} is smaller than Φ ᵢₙ (see FIG. 1b) in order not to move in the cup 11 and to be supported by the inside wall of the inner cover 5 (see FIG. 2c).

In addition, the electrode plate 12 is preferably flexible using a thin plate (the thickness is 0.1 mm in this example). Since the edge portion of the thin plate may damage the cup 11 made of a silicone material or the like. Therefore, depending on the cup material, it is necessary to partially increase the thickness of the portion touching the electrode plate 12.

It is also possible to directly attach the conductive wire included in the suction guide cord 33 to the electrode plate 12 with solder or the like without using the lead-out pipe 22. However, in that case, since it is difficult to separate the conductor 1 and the suction guide cord 33, you have to change the conductor 1 with the suction guide cord 33 at the same time.

The cup 11 has a through hole for inserting the lead-out pipe 22 (See FIG. 2c). The lead-out pipe 22 is inserted through the hole so as to be bridged over the suction portion 3 of the cup 11 and the lateral hole 21 is positioned in the suction portion 3. With this configuration, there is no air flow path in the conductive portion 2 and an air flow path is formed mainly in the suction portion 3. The tip of the lead-out pipe 22 is sealed with a stopper or the like.

With this configuration, the suction pressure of the pump 38 is transmitted to the suction portion 3 of the cup 11 and the suction force is generated in the conductor 1. Therefore, the inside of the conductive portion 2 can substantially keep it windless.

In addition, the absorber 13 is made of high conductive liquid retentive material so as to contain a large amount of conductive liquid (conductive liquid etc.). Since the absorber 13 directly touches the user's body surface, it is preferable that the absorber 13 is suitable for biological compatibility. In this example, a cellulose material using pulp, viscose, cotton and the like are preferable.

Also, the conductor 1 is necessary to be connected to the main body 31 via a suction guide cord 33 in an electrical and suction path manner. Further, the main body 31 includes at least a low-frequency output control unit 36, a main control section 37, a suction pump 38, and preferably a hot plate being a warming means 32. (See FIG. 10b). The warming means 32 need not be provided on the upper portion of the main body 31 and may be provided on the middle part of the main body or on the special base. The conductor 1 is connected to the main body 31 through the suction connection portion 34 and the electrically conductive connection unit 35.

The signal generated by the low frequency output control unit 36 is sent to the conductive portion 2 of the conductor 1 through the suction guide cord 33.

A state of attaching the conductor 1 to the body surface of the user is described with reference to FIG. 3. By pressing the conductor 1 on the body surface, the cup 11 is stuck to the body surface with deforming it. For more detail, immediately after the outer cover 5 comes into contact with the body surface, deformation of the cup 11 by suctioning is started. (See FIG. 1c). The cup 11 deforms with the outer cover 5 being compressed in the vertical direction using the slit. At this time, little suction force is generated by the suction pump 38 in the inner cover 6, but due to deformation of the outer cover 5, the tip of the surface of the inner cover 6 comes into contact with the body surface and by further being pressed against the body surface, the electrode plate 12 contained in the conductive portion 2 comes into close contact with the absorber 13. As a result, the body surface and the output control unit 36 are electrically conducted.

If the tip of the contacting portion of the outer cover 5 (Surface A) and the tip of the contacting portion of the inner cover 6 (Surface B) are provided at the same position, when the conductor 1 is adsorbed, the surface B possibly first touches the body surface not to be sucked well. For this reason, it is necessary to provide the surface B shorter than the surface A by tₐ. In addition, it is necessary to consider the thickness of the absorber 13 so that the surface B does not come out below the surface A. In this example, tₒᵤₜ is set to 16 mm, tᵢₙ is set to 12 mm, tₐ is set to 4 mm, and the thickness of the absorber 13 is set to 6 mm.

If the conductive portion 2 first attaches the body surface, there is a possibility that the outer cover 5 may be lifted by the reacting force of the absorber 13, the electrode plate 12 and the cup 11. In this case, air leakage occurs and the conductor 1 tends to drop off from the body surface. When completely falling out, the conduction of the therapeutic current disappears, but if it is fallen halfway, the current density will increase locally and there is a possibility of danger.

By setting tₒᵤₜ larger than tᵢₙ as in this example, the falling possibility of the conductor 1 can be reduced.

In addition, it is difficult to be stuck at all contacting areas using the same conductor, such as a portion having a spherical surface like a shoulder, a small curvature surface like a wrist, an unevenness surface like a face. Therefore, there may be cases in which the conductor 1 is smaller or having special shapes along the surface of the body are separately produced. Generally, the main part of electrical therapy is considered to be a substantially flat surface, like buttocks, waist, shoulder blades and back (around the shoulder blades), so in such a place, dimensions tₐ is set to about 1 mm or more.

Considering many other treatment parts including calves, thighs and the like, this part forms a tubular shape being substantially linear in one direction and orthogonal curve.

In order to attach the conductor 1 to the above-mentioned tubular portion, it is effective to grasp the conductor 1 to deform to the elliptical shape as viewed from above with the surface A and the surface B being curved as viewed orthogonally from the outlet pipe 22 in order to take a good shape along the tubular part.

Therefore, it is possible to stick to a tubular portion without providing a special contacting shape. Further, by cutting both sides of the electrode plate 12 without making it circular, the above mentioned deformation becomes easier when grasping the conductor 1.

Further, by providing the slits 7 and 8 on the wall of the outer cover 5 and the inner cover 6, deformation at the adsorption is made easier. The depth of the slit is about half of the thickness of the both covers 5, 6.

Also, deforming the conductor 1 in a bellows shape is affected by not only the hardness of the absorber 13 but also the thickness of the outer cover 5 and the inner cover 6. Experimentally, setting the wall thickness t = 1.0 to 2.0 mm makes the operation smoother. Further, the length and the thickness of the inner cover 6 are preferably in a range in which the absorber 13 cannot be an encumbrance at the suction. The inner cover 6 does not require much strength compared with the outer cover 5 so that the thickness of the inner cover 6 is made thinner than that of the outer cover 5, with t = 0.5 to 1.5 mm. With this configuration, the outer cover 5 is not obstructed by the inner cover 6 and can be smoothly adsorbed to the body surface by a suctioning operation.

Further, in the conventional bellows structure, the outer shape is larger and the unnecessarily weightier. (See FIG.13). For this reason, it weakens the attracting force of the conductor and the conductor tends to be fallen off easily. In that regard, in this example, by providing a slit in the outer cover 5 and the inner cover 6, the weight is reduced well.

In this example, the suction portion 3 and the conductive portion 2 being separated, the air and conductive liquid inside the inner cover 6 is not actively sucked at the time of suction, and the conductor 1 is not rapidly cooled. As a result, the conductor 1 is not rapidly cooled down after the conductor 1 is taken out from the warming means 32.

In particular, in the winter, the conductive liquid contained in the absorber 13 tends to be colder. In this respect, the conductor 1 of this example is excellent.

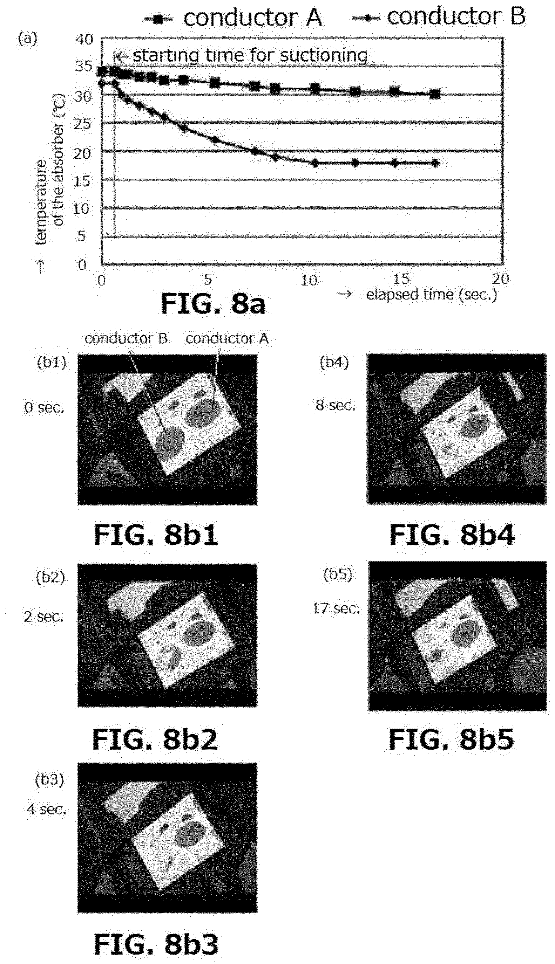

The temperature of the conductor 1 is measured as a temperature-dependent image by a thermometer and arranged in the order of FIG. 8b1 to FIG. 8b5 at each elapsed time after releasing from the warming means 32. (See FIG.8). The conductor B is the conventional conductor and the conductor A is one of example of this description. The temperature of the absorber in both conductors are plotted for comparison. (See FIG. 8a).

The measuring method is as follows. After placing the conductors of both conductor A and conductor B on the warming means 32 until they are sufficiently warmed, they are taken out from the warming means 32 and placed on a desk at room temperature, the pump 38 is operated immediately after that, the temperature change was observed. Looking at the data, it can be seen that immediately after the pump 38 is operated, the conventional conductor B is rapidly cooled down.

In the diagrams of FIG. 8b1 to FIG .8b5, the white square is the display screen of the thermometer. Inside this white square, the test environment temperature of 23 ° C is displayed in the same white as the display surface. The gray becomes darker as the temperature difference from the environmental temperature 23 ° C becomes larger.

The darkest color in the figure represents a temperature difference of ± 7 ° C. (30 ° C at high temperature and 17 ° C at low temperature).

FIG. 8b1 shows the state of the thermometer immediately after moving the conductors from the warming means 32 to the desk at room temperature. After operating the suction pump, the states 2 seconds, 4 seconds, 8 seconds, 17 seconds later are respectively figured on Figs. 8b2 ∼ 8b5. The conductor A is hardly cooled down and maintains the initial dark gray (about 30 ° C).

On the other hand, conductor B is cooled down almost to the room temperature 4 seconds later (See FIG. 8b3), and it is cooled down to about 17 °C 17 seconds later (See FIG. 8b5). It can be confirmed how it is rapidly cooled down on such a conventional structure and from this result, the difference in heat retention effect between the conductor of this example and the conventional conductor becomes clear.

In addition, when the conductor is attached to the body surface, scarring is caused by congestion, and the scar may remain on the skin for a long time even after the treatment. However, with the use of the conductor 1, it may relieve the scar due to the heat retaining effect being high.

### Example 2

Next, another example is described with FIG.4a being an external perspective view of the conductor 1 viewed from the mounting side, FIG. 4b being a front view thereof, and FIG. 4c as the A - A cross section in FIG.4b.

In conventional conductors, scarring occurs due to congestion, and the boundary of the scar appears in the vicinity of the contact portion between the cup and the body surface. If the contact portion area to the body surface of the outer cover 5 is small, the attraction force may concentrate and bite deeper into the body surface of the user, resulting in a strong scar.

In this example, the buffer portion 4 is provided at the end of the contacting portion of the outer cover 5. (See FIG. 4c). The buffer portion 4 is formed by extending the tip of the contacting portion of the outer cover 5, and is caught in an arc shape inside the cup 11. The larger the radius of the circular arc is, the greater the buffering effect is, but the larger the conductor becomes. Therefore, in this example, the arcuate radius of the buffer portion is 4 mm. In this example, the buffer portion 4 is a part of the outer cover 5. However, the buffer portion 4 may separately be retrofitted to the tip of the contacting portion of the outer cover 5 or integrally molded by insert molding. In addition, since the buffer portion 4 does not require much strength, it is possible to improve the balance during operation as the absorber 13 having a small hardness or the wall thickness of about half of the thickness of the outer cover 5.

Although the buffering portion 4 is described as an inwardly arcuate shape, the distal end of the outer cover may be branched into two directions of inward and outward. However, if only the outward facing shock absorbing part is used, it is less preferable because it may be weak against disturbance and easily falls off when adhering to a tubular diseased part.

### Example 3

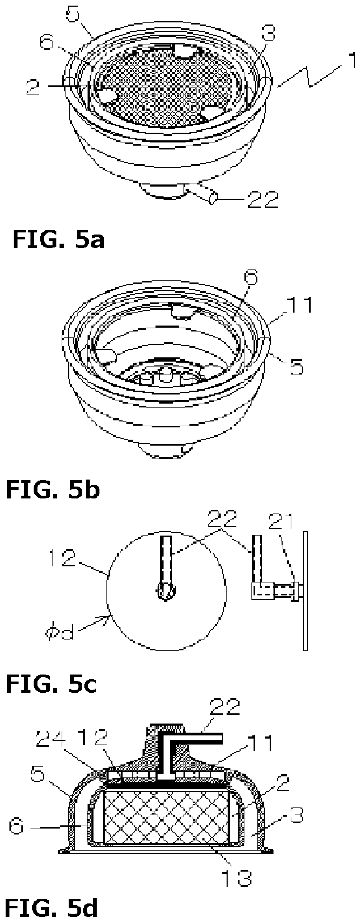

Another example will be described wit FIG. 5b being a front view thereof, FIG. 5c being a vertical cross-sectional view taken along a center line of the lead-out pipe 22, and FIG. 5d. The outer diameter Φs of the absorber 13 (See FIG.2a) containing conductive liquid and its height tₛ and the outer diameter Φd of the electrode plate 12 (See FIG. 2b) are adjusted according to the size of the inner cover 6.

The feature of this example is the pipe lateral hole 21 being provided near the upper center of the electrode plate 12.

In addition, the cup portion forming the inner cover 6 may exist separately from the cup 11 of the outer cover, when interlocked with the electrode plate 12. In addition, the three projections provided at the tip of the contacting portion of the inner cover 6 prevent the absorber 13 containing conductive liquid from falling off.

The suction operation of the cup 11 is not deformed by the slit structure but a structure in which the head of the cup 11 moves up and down. For this reason, the wall thickness of the cup 11 other than the moving part is thickened at about t = 1.5 mm to 3.5 mm. When the wall of the cup 11 is thick, since the restoring force of the cup 11 is strong, the tracking performance of the adsorption force increases, and the conductor 1 is easy to move from the user's body surface.

### Example 4

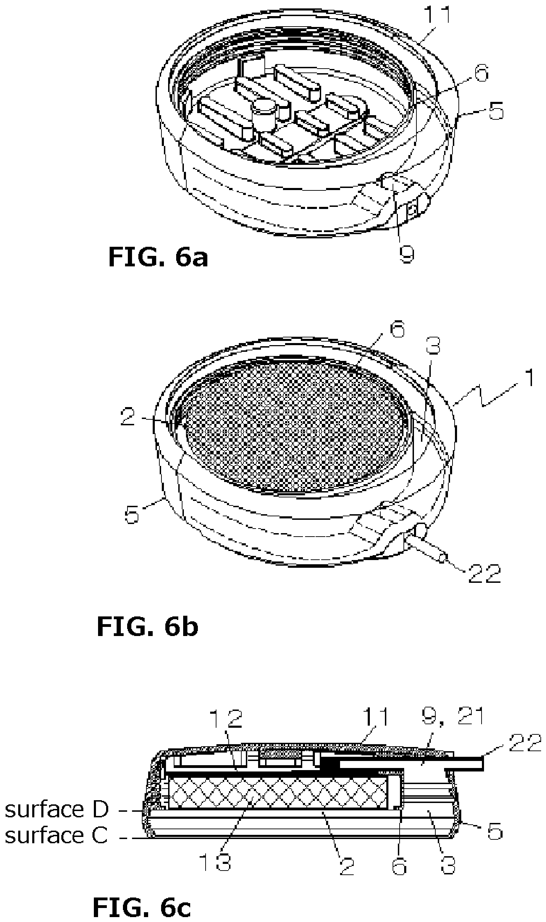

Next, another example will be described with FIG. 6a and FIG. 6b being external perspective views of the conductor 1 as viewed from the body surface side, and FIG. 6c being a sectional view taken along the lead pipe 22 as a center line. The feature of this example is that the suction portion 3 is not provided on the entire circumference of the inner part. The suction portion 3 of this example is constructed with being weighted towards one direction from the center and a space between the tip of the outer cover (the surface C in FIG. 6c) and the tip of the inner part (the surface D).

### Example 5

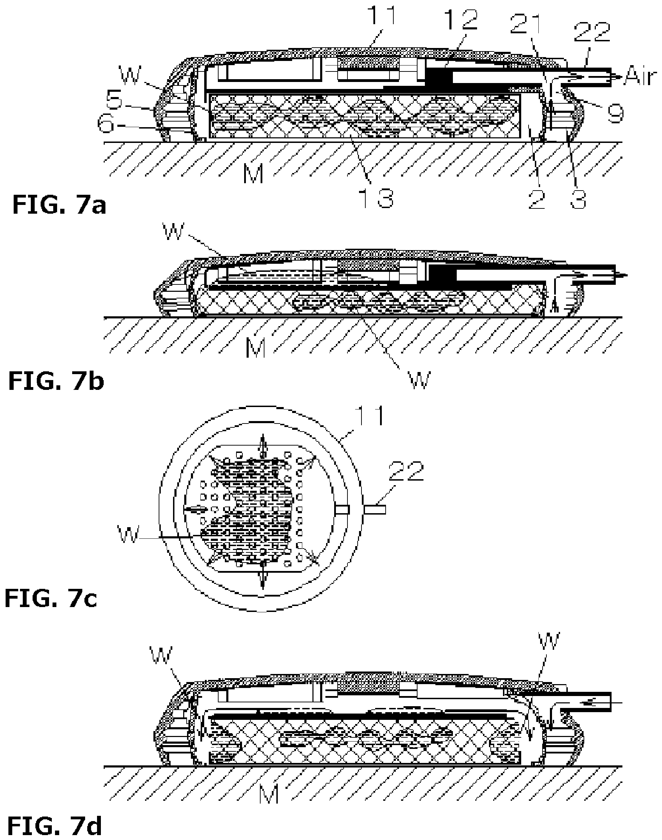

One of the principle according to the present description is described with FIG. 7 showing how the conductive liquid contained in the absorber 13. FIG. 7a is a cross-sectional view taken along the line A - A in FIG. 1b in this example and is an explanatory view showing a state before the suction force is generated in the suction portion 3. FIG. 7b is a view showing the state after the suction force is generated in the suction portion 3 and FIG. 7c is an explanatory view showing the state when the suction force disappears after the suction force is generated.

The absorber 13 containing sufficient moisture is an important part for securing stable conductivity with the electrode plate 12. In the conventional conductor, the absorber containing conductive liquid is compressed by the deformation at the time of suction, and the moisture contained in the absorber is discharged to the outside of the absorber.

In this description, the electrode portion 2 is separated from the flow path of the suction air, and the suction force of the pump 38 does not directly reach the inside of the inner cover 6, and the moisture being discharged by compression of the absorber 13 is not substantially sucked by the pump 38.

FIG. 7b shows how the conductor 1 is entirely deformed by the attraction force. The moisture (conductive liquid) discharged to the outside of the absorber 13 is temporarily stored above the electrode plate 12 (the space formed by the rib provided on the surface). (See FIG. 7c)

After finishing treatment, releasing the adsorption force restores the absorber 13 to its original shape. At that time, most of the moisture does not pass through the pores of the electrode plate 12 but reaches the outer periphery of the electrode plate 12 and is again absorbed by the absorber 13. As a result, the moisture discharged from the absorber 13 circulates inside the inner cover 6, and the reduction of moisture due to the sucking operation.

Further, the moisture contained in the absorber 13 is dried in order from the tip of the contacting portion of the absorber 13. In this example, moisture squeezed out from the absorber 13 can be replenished again from the tip of the contacting portion of the absorber 13, so that moisture can be more effectively circulated to the absorber 13.

In this example, the adverse effect of absorbing moisture and unnecessary substances discharged from the absorber 13 by the pump 38 can be suppressed. However, the unnecessary substances are likely to accumulate in the absorber 13. Therefore, for sanitation, it is preferable to frequently perform washing of the absorber 13. However, frequent washing will increase treatment costs.

As a method to reduce the unnecessary substances being accumulated in the absorber 13, suctioning it partially is also conceivable as long as the temperature of the absorber 13 is not excessively lowered. For example, a slit, at the tip of the contacting portion of the inner cover 6 to make a way for the unnecessary substances with a small amount of water to get through, can flow a part of the unnecessary substances to the suction guide cord 33 without lowering the temperature of the absorber 13 excessively.

Furthermore, by using an inexpensive nonwoven fabric or pulp (such as cotton puff) as a disposable material which can be replaced for each user, it is possible to suppress the treatment time and the treatment cost.

Especially, cotton puffs are generally preferable to use as conductive liquid absorber, because the meshes of them are so fine to prevent from scaring when they are pressed on the body surface of the human. However, it is easier to generate unnecessary substances discharged from the cotton puffs than conventional sponge type. Therefore, there is a possibility of accelerating clogging of the pipe and the pump at the suction if it is used at the conventional suction structure. However, according to the present description, since the suction structure is different, such a problem hardly occurs. Therefore, it can be used as a conductive liquid absorber disposably for each user.

## Claims

1. An electrotherapeutic device for muscle stimulation, comprising:
a main body (31) configured to generate an electric current; and
a conductor (1) connected to the main body (31) via a suction guide cord (33) in both an electrical and a suction path manner, and having a conductive portion (2) electrically connected to the body surface of the user via an absorber (13) containing conductive liquid, and a suction portion (3) configured to adhere the conductor (1) to the body surface of the user,
**characterized in that** the conductor (1) further comprises:
an inner cover (6) covering the conductive portion (2); and
an outer cover (5) disposed outside the inner cover (6),
wherein each of the inner cover (6) and the outer cover (5) has a contacting portion configured to contact with the body surface of the user, and the suction portion (3) is disposed between the inner cover (6) and the outer cover (5) while keeping it separated from the conductive portion (2).

2. The electrotherapeutic device in accordance with claim 1, wherein the suction portion (3) is connected with a suction pump (38) via the suction guide cord (33).

3. The electrotherapeutic device in accordance with any one of claims 1 or 2, wherein the electric current generated by the main body (31) is a low frequency current.

4. The electrotherapeutic device in accordance with any one of claims 1 to 3, wherein the conductive liquid contained in the absorber (13) is water.

5. The electrotherapeutic device in accordance with any one of claims 1 to 4, wherein the absorber (13) is made of nonwoven fabric or pulp, and configured to be disposable for each user.

6. The electrotherapeutic device in accordance with any one of claims 1 to 5, wherein both the outer cover (5) and the inner cover (6) have a cup-shaped portion.

7. The electrotherapeutic device in accordance with any one of claims 1 to 6, wherein the tip of the contacting portion of the inner cover (6) is shorter than that of the outer cover (5).

8. The electrotherapeutic device in accordance with any one of claims 1 to 7, wherein the tip of the contacting portion of the inner cover (6) is softer than that of the outer cover (5).

9. The electrotherapeutic device in accordance with any one of claims 1 to 8, wherein walls formed of both the inner cover (6) and outer cover (5) toward the body surface have recesses or slits to deform them easily toward the body surface.

10. The electrotherapeutic device in accordance with any one of claims 1 to 9, wherein the wall forming the inner cover (6) is provided vertically against the body surface of the user, and that of the outer cover (5) is provided with an angle 45 ° or less, preferably 5 to 15°, against the inner cover (6).

11. The electrotherapeutic device in accordance with any one of claims 1 to 10; further comprising:
an electrode plate (12) being in contact with the absorber (13),
wherein the electrode plate (12) is provided with fine holes (23) and the inner cover (6) has a rib on the part of the inner bottom surfacing the electrode plate (12).

12. The electrotherapeutic device in accordance with any one of claims 1 to 11; further comprising:
a lead-out pipe (22) making an electrical and suctioning path between the conductor (1) and the suction guide cord (33),
wherein the lead out pipe (22) connects with the suction portion (3) by using a lateral hole (21) of it.

## Patentansprüche

1. Elektrotherapeutische Vorrichtung für Muskelstimulation, umfassend:
einen Hauptkörper (31), der konfiguriert ist, um einen elektrischen Strom zu erzeugen; und
einen Leiter (1), der mit dem Hauptkörper (31) über eine Saugführungsleitung (33) auf eine Art und Weise sowohl eines elektrischen als auch eines saugfähigen Wegs verbunden ist, und aufweisend einen leitfähigen Abschnitt (2), der mit der Körperoberfläche des Benutzers über einen Absorber (13) elektrisch verbunden ist, der leitfähige Flüssigkeit enthält, und einen Saugabschnitt (3), der konfiguriert ist, um den Leiter (1) an die Körperoberfläche des Benutzers zu binden, **dadurch gekennzeichnet, dass** der Leiter (1) ferner umfasst:
eine Innenabdeckung (6), die den leitfähigen Abschnitt (2) abdeckt; und
eine Außenabdeckung (5), die außerhalb der Innenabdeckung (6) angeordnet ist, wobei jede der Innenabdeckung (6) und der Außenabdeckung (5) einen Berührungsabschnitt aufweist, der konfiguriert ist, um die Körperoberfläche des Benutzers zu berühren, und der Saugabschnitt (3) zwischen der Innenabdeckung (6) und der Außenabdeckung (5) angeordnet ist, während er von dem leitfähigen Abschnitt (2) getrennt gehalten wird.

2. Elektrotherapeutische Vorrichtung nach Anspruch 1, wobei der Saugabschnitt (3) mit einer Saugpumpe (38) über die Saugführungsleitung (33) verbunden ist.

3. Elektrotherapeutische Vorrichtung nach einem der Ansprüche 1 oder 2, wobei der elektrische Strom, der durch den Hauptkörper (31) erzeugt wird, ein Niedrigfrequenzstrom ist.

4. Elektrotherapeutische Vorrichtung nach einem der Ansprüche 1 bis 3, wobei die leitfähige Flüssigkeit, die in dem Absorber (13) enthalten ist, Wasser ist.

5. Elektrotherapeutische Vorrichtung nach einem der Ansprüche 1 bis 4, wobei der Absorber (13) aus Vliesstoff oder Zellstoff hergestellt ist, und konfiguriert ist, um für jeden Benutzer wegwerfbar zu sein.

6. Elektrotherapeutische Vorrichtung nach einem der Ansprüche 1 bis 5, wobei sowohl die Außenabdeckung (5) als auch die Innenabdeckung (6) einen becherförmigen Abschnitt aufweisen.

7. Elektrotherapeutische Vorrichtung nach einem der Ansprüche 1 bis 6, wobei die Spitze des Berührungsabschnitts der Innenabdeckung (6) kürzer als die der Außenabdeckung (5) ist.

8. Elektrotherapeutische Vorrichtung nach einem der Ansprüche 1 bis 7, wobei die Spitze des Berührungsabschnitts der Innenabdeckung (6) weicher als die der Außenabdeckung (5) ist.

9. Elektrotherapeutische Vorrichtung nach einem der Ansprüche 1 bis 8, wobei die Wände, die sowohl aus der Innenabdeckung (6) als auch aus der Außenabdeckung (5) ausgebildet sind, in Richtung der Körperoberfläche Vertiefungen oder Schlitze aufweisen, um sie in Richtung der Körperoberfläche leicht umzuformen.

10. Elektrotherapeutische Vorrichtung nach einem der Ansprüche 1 bis 9, wobei die Wand, die die Innenabdeckung (6) ausbildet, senkrecht gegen die Körperoberfläche des Benutzers vorgesehen ist, und diejenige der Außenabdeckung (5) mit einem Winkel von höchstens 45°, vorzugsweise 5 bis 15°, gegen die Innenabdeckung (6) vorgesehen ist.

11. Elektrotherapeutische Vorrichtung nach einem der Ansprüche 1 bis 10;
ferner umfassend:
eine Elektrodenplatte (12), die den Absorber (13) berührt, wobei die Elektrodenplatte (12) mit feinen Löchern (23) versehen ist und die Innenabdeckung (6) eine Rippe an dem Teil des Innenbodens aufweist, der auf der Elektrodenplatte (12) aufliegt.

12. Elektrotherapeutische Vorrichtung nach einem der Ansprüche 1 bis 11;
ferner umfassend:
ein Entladungsrohr (22), das einen elektrischen und einen saugfähigen Weg zwischen dem Leiter (1) und der Saugführungsleitung (33) herstellt, wobei das Entladungsrohr (22) sich mit dem Saugabschnitt (3) unter Verwendung eines seitlichen Lochs (21) davon verbindet.

## Revendications

1. Dispositif électrothérapeutique pour la stimulation des muscles, comprenant :
un corps principal (31) conçu pour produire un courant électrique ; et
un conducteur (1) connecté tant électriquement que par effet de ventouse au corps principal (31) par l'intermédiaire d'un cordon guide de ventouse (33), et ayant une partie conductrice (2) connectée électriquement à la surface du corps de l'utilisateur par l'intermédiaire d'un absorbeur (13) contenant un liquide conducteur, et une partie ventouse (3) conçue pour faire adhérer le conducteur (1) à la surface du corps de l'utilisateur,
**caractérisé en ce que** le conducteur (1) comprend en outre :
un couvercle intérieur (6) recouvrant la partie conductrice (2) ; et
un couvercle extérieur (5) disposé à l'extérieur du couvercle intérieur (6),
le couvercle intérieur (6) et le couvercle extérieur (5) ayant une partie de contact conçue pour entrer en contact avec la surface du corps de l'utilisateur, et la partie ventouse (3) étant disposée entre le couvercle intérieur (6) et le couvercle extérieur (5) tout en la maintenant séparée de la partie conductrice (2).

2. Dispositif électrothérapeutique selon la revendication 1, la partie ventouse (3) étant raccordée à une pompe aspirante (38) par l'intermédiaire du cordon de guide de ventouse (33).

3. Dispositif électrothérapeutique selon l'une quelconque des revendications 1 ou 2, le courant électrique produit par le corps principal (31) étant un courant basse fréquence.

4. Dispositif électrothérapeutique selon l'une quelconque des revendications 1 à 3, le liquide conducteur contenu dans l'absorbeur (13) étant de l'eau.

5. Dispositif électrothérapeutique selon l'une quelconque des revendications 1 à 4, l'absorbeur (13) étant fabriqué en tissu non tissé ou de pâte, et étant à usage unique pour chaque utilisateur.

6. Dispositif électrothérapeutique selon l'une quelconque des revendications 1 à 5, le couvercle extérieur (5) et le couvercle intérieur (6) ayant une forme de cupule.

7. Dispositif électrothérapeutique selon l'une quelconque des revendications 1 à 6, la pointe de la partie de mise en contact du couvercle intérieur (6) étant plus courte que celle du couvercle extérieur (5).

8. Dispositif électrothérapeutique selon l'une quelconque des revendications, la pointe de la partie de mise en contact du couvercle intérieur (6) étant plus souple que celle du couvercle extérieur (5).

9. Dispositif électrothérapeutique selon l'une quelconque des revendications 1 à 8, des parois formées du couvercle intérieur (6) et du couvercle extérieur (5) en direction de la surface du corps présentant des évidements ou des fentes pour se déformer facilement en direction de la surface du corps.

10. Dispositif électrothérapeutique selon l'une quelconque des revendications 1 à 9, la paroi formant le couvercle intérieur (6) étant disposée verticalement contre la surface du corps de l'utilisateur, et celle du couvercle extérieur (5) étant dotée d'un angle de 45° au plus, de préférence compris entre 5 et 15°, contre le couvercle intérieur (6).

11. Dispositif électrothérapeutique selon l'une quelconque des revendications 1 à 10, comprenant en outre :
une plaque d'électrode (12) en contact avec l'absorbeur (13),
la plaque d'électrode (12) étant pourvue de trous minces (23) et le couvercle intérieur (6) ayant une rainure sur la partie du fond intérieur recouvrant la plaque d'électrode (12).

12. Dispositif électrothérapeutique selon l'une quelconque des revendications 1 à 11, comprenant en outre :
une conduite de sortie (22) établissant un chemin électrique et un effet de ventouse entre le conducteur (1) et le corde de guide de ventouse (33),
la conduite de sortie (22) étant reliée à la partie ventouse (3) par un trou latéral (21) en son sein.
